# FASCICULE DE BREVET EUROPEEN

(11) **EP 0 729 760 B1**
(45) Date de publication et mention de la délivrance du brevet: **12.12.2001**
(21) Numéro de dépôt: 96420067.9
(22) Date de dépôt: 29.02.1996
(51) Int. Cl.: A61L 2/26, A01N 1/02

(54) **Boîtier protecteur d'un conditionnement pour stérilisation**
Schutzbehälter für Sterilisationsgefä
Protective case for sterilisation package

(30) Priorité: 01.03.1995 FR 9502656
(43) Date de publication de la demande: 04.09.1996
(73) Titulaire: MERCK BIOMATERIAL FRANCE, 01800 Charnoz (FR); Chiron, Philippe Charles, 31100 Toulouse (FR)
(72) Inventeur: Chiron, Philippe Charles, 31100 Toulouse (FR); Collomb, Jean, 26800 Portes Les Valence (FR); Picault, Charles, 69110 Sainte-Foy-Les-Lyon (FR)
(74) Mandataire: Ropital-Bonvarlet, Claude

(56) Documents cités:
- EP-A- 0 152 544
- EP-A- 0 584 484
- WO-A-87/05520

## Description

La présente invention est relative au domaine de la conservation de matières et de matériels organiques, en vue de permettre leur utilisation ultérieure en toute sécurité.

L'invention peut concerner différents domaines d'application, tels que celui de la conservation de produits alimentaires ou de la conservation de matériels organiques réutilisables dans le domaine pharmaceutique, médical, biologique etc.

La conservation de matières ou matériels organiques a déjà fait l'objet de publications relativement nombreuses faisant état de méthodes de conservation variées.

Il peut être cité, à titre indicatif, l'incubation telle qu'enseignée par la demande **EP-A-0 584 484,** la cryoconservation, la lyophilisation, ainsi que la stérilisation.

La première méthode n'apparaît pas de nature à satisfaire les exigences de suppression des risques de contamination résiduelle de la matière.

Les deux méthodes suivantes présentent l'inconvénient de faire intervenir des procédés lourds et relativement coûteux qui, par ailleurs, ne fournissent pas une sécurité absolue quant aux risques de contamination résiduelle par des micro-organismes ou par des virus.

Le procédé de stérilisation peut connaître différentes méthodes, telles que l'application de rayonnements, l'application d'oxyde d'éthylène ou la stérilisation par chaleur humide.

L'application de rayonnements, du type X, Gamma ou bêta, implique un matériel important et de conduite délicate. Par ailleurs, de tels rayonnements sont à l'origine d'une altération des propriétés des matières organiques dont la réutilisation peut être de la sorte, en partie gênée, voire entravée. De plus, le coût de mise en oeuvre d'une telle méthode est élevé.

La stérilisation par l'oxyde d'éthylène possède l'inconvénient de représenter pour le personnel un risque, soit direct, soit par l'intermédiaire des produits de réaction secondaire, tels que l'éthylène-glycol et l'éthylène-chloridrine, produits réputés toxiques.

La stérilisation par chaleur humide apparaît une technique intéressante, en raison de la possibilité qu'elle offre d'être menée à bien, à partir de moyens techniques relativement simples, avec une dépense d'énergie relativement faible.

Le problème qui se pose toutefois pour la mise en oeuvre d'une telle méthode est de disposer d'un matériel qui permette de mener à bien le processus de stérilisation par voie humide, tel qu'il est connu, au moyen d'un autoclave et d'un processus d'application séquentielle de températures bien déterminées et qui permette, de surcroît, d'assurer une conservation de la matière ou du matériel stérilisé dans des conditions de sécurité absolues rendant possible un stockage, un transport, ainsi qu'une possible réutilisation dans des conditions optimales.

Un autre problème est celui de préserver la matière ou le matériel organique devant être stérilisé, en étant assuré visuellement que la stérilisation intervenue correspond exactement au processus opératoire ayant été conduit.

Il peut être estimé qu'à l'heure actuelle, la technique connue ne fournit pas de matériel propre à répondre aux exigences ci-dessus.

L'objet de l'invention est de contester la présente lacune en proposant un boîtier protecteur de stérilisation et de conservation qui permette d'assumer la double fonction ci-dessus pour un conditionnement de stérilisation, de conception connue, de telle manière que la fonction de stérilisation puisse intervenir dans des conditions optimales, et que la fonction de stockage, manipulation et transport puisse être assumée en assurant une protection efficace du conditionnement contre les risques d'ouverture intempestive, mais aussi contre les chocs, les coups, voire les chutes, qui peuvent résulter des opérations de manipulation, de stockage, de transport.

Pour atteindre les objectifs ci-dessus, le boîtier protecteur de stérilisation et de conditionnement de matière ou matériel organique contenu dans un conditionnement formé par un bocal et par une capsule de fermeture étanche est caractérisé en ce qu'il comprend
- un corps de boîtier apte à recevoir et immobiliser un conditionnement,
- un couvercle de boîtier adaptable sur le corps et formant intérieurement au moins une protubérance de coopération avec la capsule du conditionnement,
- des moyens de liaison amovible conçus pour soumettre le corps et le couvercle, lors de la fermeture du boîtier, à un rapprochement relatif axial,
- et au moins une ouverture prévue dans le corps et/ou le couvercle pour mettre en relation le volume interne du boîtier fermé avec le milieu ambiant.

L'invention a encore pour objet un ensemble de stérilisation et de conservation de matière ou matériel organique caractérisé en ce qu'il se compose d'un conditionnement comprenant un bocal et une capsule perforable de fermeture étanche et d'un boîtier protecteur selon l'une des revendications 1 à 8.

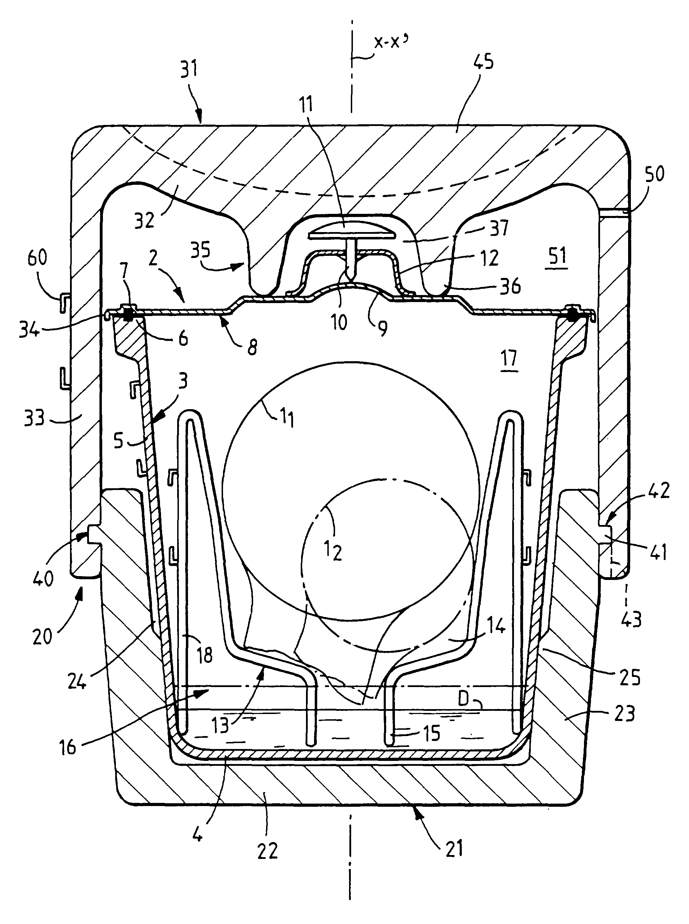

Diverses autres caractéristiques ressortent de la description faite ci-dessous en référence au dessin annexé qui montre, à titre d'exemple non limitatif, une forme de réalisation de l'objet de l'invention.

La **figure unique** est une coupe-élévation d'une forme de réalisation de l'objet de l'invention.

Le boîtier protecteur conforme à l'invention est conçu pour permettre la stérilisation et la conservation de matière ou matériel organique tel que **1,** contenu dans un conditionnement tel que **2.** La matière **1** est représentée sous la forme de transplants osseux, par exemple de têtes de fémur, dont deux tailles sont illustrées et désignées par les références **1**_{**1**} et **1**_{**2**}**.** Il doit être considéré que l'objet de l'invention peut s'appliquer à toute autre matière ou matériel organique et peut trouver une utilisation dans le domaine des matières alimentaires ou des tissus organiques relevant du domaine pharmaceutique, biomédical etc.

Dans le but d'assurer la stérilisation et la conservation d'un matériel tel que **1,** le conditionnement **2** comprend un bocal **3** de forme tronconique, ouvert vers le haut à partir d'un fond **4.** Le bocal **3** comprend une paroi périphérique **5** dont le bord supérieur définit une portée annulaire **6** sur laquelle prend appui, par l'intermédiaire d'un joint **7,** une capsule **8** présentant, dans sa partie centrale coïncidant avec l'axe **xx'** de révolution du bocal, un bulbe **9** destiné à être percé par l'intermédiaire d'un perforateur **10.** Au sens de l'invention, le perforateur **10** est constitué par l'aiguille d'un bouton **11** qui est porté par un pontet **12** s'élevant à partir de la capsule **8.**

De manière à permettre la stérilisation et la conservation de la matière ou du matériel **1,** le bocal contient intérieurement un gobelet ou une crépine **13** délimitant un logement **14** pour la réception du matériel **1.** Le gobelet ou la crépine **13** possède en partie basse des pattes **15** destinées à prendre appui sur le fond **4,** pour délimiter un compartiment **16** apte à recevoir une dose **D** d'un produit liquide approprié apte à fournir une vapeur saturée lors de l'engagement et du déroulement du processus de stérilisation.

Le gobelet **13** ou la crépine possède un fond perforé ou autrement ajouré, de manière à établir une communication entre le compartiment **16** et le logement **14** qui est ouvert en partie supérieure pour communiquer également avec le volume interne **17** délimité par le bocal **3.** La périphérie du gobelet **13** est pourvue de pattes extérieures **18** qui sont prévues pour coopérer pour partie avec le fond **4** et pour partie avec la paroi périphérique **5,** de manière à assurer élastiquement le centrage du gobelet **13** à l'intérieur du volume **17** et préserver la matière ou le matériel **1** de tout contact avec la paroi périphérique **5.**

Un conditionnement du type ci-dessus est prévu pour être soumis à une phase de stérilisation faisant intervenir, d'une manière connue, des séquences de montée en température et de mise sous vide, de manière à établir une vapeur humide saturée dans le volume **17,** selon des conditions de température voisines de 120° C et une durée d'exposition de l'ordre de 20 minutes.

Pour mener à bien un tel procédé de stérilisation, l'invention préconise de mettre en oeuvre un boîtier protecteur désigné dans son ensemble par la référence **20.** Le boîtier protecteur comprend un corps de boîtier **21** constitué par un fond **22** bordé par une paroi périphérique **23,** de manière à délimiter un logement **24** apte à recevoir le conditionnement **2** et, plus particulièrement, le bocal **3.** Dans l'objectif de l'invention, le logement **24** est pourvu de moyens qui sont propres à assurer l'immobilisation transversale et axiale du conditionnement **2,** tout en maintenant de préférence dans cet état le fond **4** du bocal **3** à distance du fond **22** du corps de boîtier **21.** De tels moyens désignés par la référence **25** sont, par exemple, constitués par des surépaisseurs au moins locales, de préférence mais non exclusivement axiales, formées en saillie par la face interne de la paroi périphérique **23,** de manière à présenter une convergence en direction du fond **2.** De cette manière, le bocal **3** peut être inséré dans le logement **24** pour y être coincé dans un état d'immobilisation axiale et transversale dans lequel, comme cela apparaît au dessin, le fond **4** est placé à distance du fond **22.**

Pour assumer cette fonction, ainsi que celle de protection, le corps de boîtier **21** peut être réalisé en toute matière acceptant une certaine déformation élastique ou plastique. A titre d'exemple, la matière constitutive du corps de boîtier **21** peut être du polystyrène expansé ou une matière analogue ayant fait l'objet d'un développement en mousse plus ou moins compacte à cellules ouvertes ou fermées.

Selon une autre disposition de **l'**invention, le boîtier protecteur **20** se compose également d'un couvercle de boîtier **31** qui est réalisé à la manière d'un bocal renversé pour comporter un dessus **32** prolongé par une jupe **33** apte à emboîter la paroi périphérique **23.** La jupe **33** présente une hauteur telle qu'elle enveloppe la partie du conditionnement **2** s'élevant au-delà du bord supérieur de la paroi périphérique **23,** ainsi qu'une section de passage qui est supérieure au diamètre de la capsule **8.** De la sorte, l'emboîtement du couvercle de boîtier **31** laisse subsister un espace ou intervalle **34** entre la paroi périphérique interne de la jupe **33** et le bord périphérique de la capsule **8.** Le couvercle de boîtier **31** est réalisé en une matière identique ou similaire à celle du corps de boîtier **21** pour assumer une même fonction de protection.

Le dessus **32** comporte sur sa face intérieure une protubérance **35** qui est destinée à prendre appui sur la capsule **8** dans l'état fermé résultant de l'association du corps de boîtier **21** et du couvercle de boîtier **31.** La protubérance **35** est de préférence constituée par une nervure annulaire **36** concentrique à l'axe de révolution **xx'** et qui délimite une cavité **37** dite d'emboîtement et de protection du bouton **11** et du pontet **12.** La cavité **37** est concue pour accepter l'insertion du perforateur **16** quelque soit sa forme de réalisation et dans son état inactif de non perforation, tel qu'il résulte du dessin annexé, lorsque la nervure **36** prend appui sur la capsule **8.**

Le boîtier protecteur décrit ci-dessus est complété par la présence de moyens **40** de liaison amovible entre le couvercle **31** et le corps **21.** Les moyens **40** sont dans tous les cas choisis de manière à soumettre le corps **21** et le couvercle **31,** lors de la fermeture du boîtier, à un rapprochement axial relatif ayant pour effet d'appuyer ou de presser la protubérance **35** sur la capsule **8** en appui contre le bocal **3** immobilisé dans le corps **21.** A titre d'exemple illustratif et non limitatif, les moyens **40** peuvent faire intervenir des rampes ou des filets hélicoïdaux **41** destinés à coopérer avec des rainures **42** dans lesquelles les rampes **41** peuvent être engagées par passage dans des dégagements **43.**

Les moyens **40** assurent une liaison amovible par vissage ou analogue et, à cette fin, il est avantageux de conformer le dessus **32** pour lui faire comporter une poignée ou analogue **45** qui est ménagée sous la forme d'une poutre transversale saillante par rapport au dessus **32,** mais s'inscrivant dans l'enveloppe du bocal que délimite le couvercle de boîtier **31.**

Selon l'invention, le boîtier comporte encore au moins une ouverture, lumière ou passage, **50** ménagé dans le corps de boîtier **21** ou dans le couvercle de boîtier **31** et, de préférence encore, dans la jupe **33,** de manière à établir une communication permanente entre le milieu ambiant et le volume interne **51** qui est ménagé autour du conditionnement **2** par l'association du corps **21** et du couvercle **31** formant ensemble le boîtier protecteur **20.**

La mise en oeuvre de l'objet de l'invention pour une opération de stérilisation et de conservation, consiste à charger le bocal **3** avec la dose **D,** puis avec la matière **1** posée dans le logement **14** du gobelet **13.** Le bocal **3** est ensuite mis en place dans le logement **24** pour y être immobilisé transversalement et axialement. La capsule **8** est ensuite placée sur la plage périphérique **6,** pour permettre la mise en place à la manière d'une coiffe du couvercle **31** amené, par la coopération des rampes **41** et des rainures **42,** à exercer par la protubérance **35** l'action de pressage maintenant la capsule **8** sur le bocal **3.** Dans cet état, l'ensemble peut être manipulé et soumis à une opération de stérilisation sans aucun risque, étant donné que le conditionnement **2** est protégé contre les coups, les chocs et les détériorations, que la matière **1** est disposée à l'intérieur du bocal **3** fermé et que la capsule **8** est maintenue en place par l'intermédiaire du couvercle **31.**

Le déroulement d'un procédé de stérilisation permet, par l'intermédiaire des ouvertures **50,** la mise sous vide du volume **51** et du volume **17,** ainsi que la montée en température produisant ou générant dans le volume **17** la vapeur humide saturée occupant tout le volume utile, ainsi que le logement **14** pour soumettre la matière **1** à la phase de stérilisation connue.

En fin de stérilisation, la capsule **8** est appliquée par la pression atmosphérique sur le bocal **3** qui est ainsi fermé de façon étanche dans un état de sécurité propre à assurer la conservation de la matière **1.** En effet, le boîtier protecteur assure une protection efficace des éléments constitutifs du conditionnement **2** et protège la capsule **8** contre tout risque de détérioration ou d'action de poussée ou de traction notamment appliquée sur le bord **34** et susceptible de conduire à une ouverture intempestive.

Le boîtier protecteur permet donc la manipulation de l'ensemble, le stockage, le transport, sans aucun risque de détérioration et de dénaturation de l'état de stérilisation intervenu.

Le boîtier protecteur permet aussi, le cas échéant et en cas de besoin, de porter une appréciation visuelle sur le conditionnement **2** et son contenu, étant donné qu'il suffit de dévisser ou de déplacer relativement par rotation sur l'axe **xx',** le couvercle **31** et le corps **21** pour permettre une ouverture fournissant un accès visuel en raison de la matière constitutive translucide au moins, ou le plus généralement transparente, retenue pour constituer le bocal **3.**

Lorsqu'il convient de rendre possible un accès à la matière **1,** en vue de son utilisation, il suffit, après enlèvement du couvercle **31,** d'exercer une action de poussée sur le bouton **11** pour que le perforateur **10** perce la capsule **8** au droit du bulbe **9** et rétablisse la pression atmosphérique dans le volume **17.**

Le couvercle ou le boîtier **21** peut comporter en surface extérieure, des moyens tels que **60,** permettant l'insertion et le maintien de moyens d'identification du contenu et des conditions de stérilisation imposées à l'ensemble constitué par le conditionnement **2** et le boîtier **20.**

## Revendications

1. Boîtier protecteur de stérilisation et de conservation de matière ou matériel organique contenu dans un conditionnement formé par un bocal **(3)** et par une capsule **(8)** de fermeture étanche
**caractérisé en ce qu'**il comprend
- un corps de boîtier **(21)** apte à recevoir et immobiliser ledit conditionnement,
- un couvercle de boîtier **(31)** adaptable sur le corps et formant intérieurement au moins une protubérance **(35)** qui est destinée à prendre appui sur la capsule de conditionnement,
- des moyens **(40)** de liaison amovible conçus pour soumettre le corps et le couvercle, lors de la fermeture du boîtier, à un rapprochement relatif axial, ayant pour effet d'appuyer ou de presser la protubérance **(35)** sur la capsule **(8)** en appui contre le bocal **(3)** immobilisé dans le corps **(21)**.
- et au moins une ouverture **(50)** prévue dans le corps et/ou le couvercle pour mettre en relation le volume interne **(17)** du boîtier fermé avec le milieu ambiant.

2. Boîtier protecteur selon la revendication **1, caractérisé en ce que** le corps de boîtier comprend un fond **(22)** et une paroi périphérique **(23)** délimitant un logement **(24)** ouvert de réception et d'immobilisation d'un conditionnement.

3. Boîtier protecteur selon la revendication **1** ou **2, caractérisé en ce que** le corps **(21)** de boîtier comprend des moyens **(25)** d'immobilisation transversale et axiale d'un conditionnement à distance du fond **(22)** du corps de boîtier.

4. Boîtier protecteur selon la revendication **3, caractérisé en ce que** les moyens d'immobilisation **(25)** sont formés par des surépaisseurs au moins locales présentées par la face interne de la paroi périphérique **(23)** du corps de boîtier.

5. Boîtier protecteur selon la revendication **1, caractérisé en ce que** le couvercle **(31)** de boîtier est réalisé en forme de coiffe et comprend un dessus **(32)** bordé par une jupe **(33)** destinée à entourer à distance le conditionnement **(2)** et sa capsule **(8).**

6. Boîtier protecteur selon la revendication **1** ou **5, caractérisé en ce que** le couvercle **(31)** de boîtier comporte sur la face interne de son dessus **(32)** une protubérance **(35)** constituée par une nervure annulaire **(36)** destinée, lors de la fermeture du boîtier, à presser la capsule sur le bocal et à emboîter ce dernier dans le logement **(24)** du corps du boîtier.

7. Boîtier protecteur selon la revendication **6, caractérisé en ce que** la nervure annulaire **(36)** est concentrique à l'axe de révolution **(xx')** de la jupe **(33)** et délimite une cavité **(37)** d'emboîtement et de protection d'un perforateur de capsule.

8. Boîtier protecteur selon la revendication **1, caractérisé en ce que** les moyens de liaison **(40)** sont du type à filet hélicoïdal et **en ce que** le dessus **(32)** du couvercle forme une poignée **(45)** de préhension et d'entraînement en rotation du couvercle par rapport au corps.

9. Ensemble de stérilisation et de conservation de matière ou matériel organique, **caractérisé en ce qu'**il se compose d'un conditionnement **(2)** comprenant un bocal **(3))** et une capsule **(8)** perforable de fermeture étanche et d'un boîtier protecteur **(20)** selon l'une des revendications **1** à **8.**

10. Ensemble selon la revendication **9, caractérisé en ce que** la capsule du conditionnement porte un perforateur **(10, 11)** qui est disposé dans la cavité **(37)** d'emboîtement et de protection délimitée par la protubérance **(35)** du couvercle du boîtier.

## Patentansprüche

1. Schutzbehälter zur Sterilisierung und Konservierung von organischem Stoff oder Material, das in einer von einem Gefäß (3) und einer dichten Verschlusskapsel (8) gebildeten Verpackung enthalten ist,
**dadurch gekennzeichnet, dass** er folgendes umfasst:
- einen Korpus (21), der diese Verpackung aufzunehmen und zu fixieren vermag,
- einen Behälterdeckel (31), der an den Korpus angepasst werden kann und innen wenigstens eine Vorwölbung (35) bildet, die dazu bestimmt ist, auf der Verschlusskapsel aufzuliegen,
- lösbare Verbindungsmittel (40), die so konzipiert sind, dass sie Korpus und Deckel beim Schließen des Behälters sich einander axial annähern lassen, wobei hierdurch die Vorwölbung (35) an die Kapsel (8) angelegt oder auf sie aufgedrückt wird, die wiederum auf dem im Korpus (21) fixierten Gefäß (3) aufliegt,
- sowie wenigstens eine in dem Korpus und/oder dem Deckel vorgesehene Öffnung (50), um zwischen dem inneren Rauminhalt (17) des geschlossenen Behälters und der äußeren Umgebung eine Verbindung herzustellen.

2. Schutzbehälter nach Anspruch 1,
**dadurch gekennzeichnet, dass** der Behälterkorpus einen Boden (22) und eine periphere Wand (23) aufweist, welche einen offenen Sitz (24) zur Aufnahme und Fixierung einer Verpackung abgrenzen.

3. Schutzbehälter nach Anspruch 1 oder 2,
**dadurch gekennzeichnet, dass** der Behälterkorpus (21) Mittel (25) zur quergerichteten und axialen Fixierung einer Verpackung mit Abstand zum Boden (22) des Behälterkorpus umfasst.

4. Schutzbehälter nach Anspruch 3,
**dadurch gekennzeichnet, dass** die Fixierungsmittel (25) durch wenigstens lokale Verdickungen gebildet werden, die die Innenseite der peripheren Wand (23) des Behälterkorpus aufweist.

5. Schutzbehälter nach Anspruch 1,
**dadurch gekennzeichnet, dass** der Behälterdeckel (31) in Form einer Kappe ausgeführt ist und ein Oberteil (32) aufweist, das von einer Schürze (33) umrandet ist, die dazu bestimmt ist, die Verpackung (2) und deren Kapsel (8) von diesen beabstandet zu umgeben.

6. Schutzbehälter nach Anspruch 1 oder 5,
**dadurch gekennzeichnet, dass** der Behälterdeckel (31) an der Innenseite seines Oberteils (32) eine Vorwölbung (35) aufweist, die von einer ringförmigen Rippe (36) gebildet wird, die beim Schließen des Behälters die Kapsel auf das Gefäß drücken und dieses in den Sitz (24) des Behälterkorpus einfügen soll.

7. Schutzbehälter nach Anspruch 6,
**dadurch gekennzeichnet, dass** die ringförmige Rippe (36) zur Drehachse (xx') der Schürze (33) konzentrisch ist und einen Hohlraum (37) zum Einführen und zum Schutz einer Vorrichtung zum Perforieren der Kapsel begrenzt.

8. Schutzbehälter nach Anspruch 1,
**dadurch gekennzeichnet, dass** die Verbindungsmittel (40) in der Art eines wendelförmigen Gewindes ausgeführt sind und das Oberteil (32) des Deckels einen Griff (45) zum Fassen und Drehen des Deckels bezüglich des Korpus bildet.

9. Einheit zur Sterilisierung und Konservierung von organischem Stoff oder Material,
**dadurch gekennzeichnet, dass** sie sich aus einer Verpackung (2) mit einem Gefäß (3) und einer perforierbaren Kapsel (8) zum dichten Verschließen und einem Schutzbehälter (20) gemäß einem der Ansprüche 1 bis 8 zusammensetzt.

10. Einheit nach Anspruch 9,
**dadurch gekennzeichnet, dass** die Verpackungskapsel eine Perforationsvorrichtung (10, 11) aufweist, welche in dem Hohlraum (37) angeordnet ist, der zum Einführen und zum Schutz der Vorrichtung vorgesehen und durch die Vorwölbung (35) des Behälterdeckels begrenzt ist.

## Claims

1. A protective box for sterilising and preserving organic matter or material contained in a packing formed by a jar (3) and a hermetically closing capsule (8),
**characterised in that** it comprises
- a box body (21) adapted to receive and immobilize said packing;
- a box lid (31) adaptable on the body and forming, internally, at least one protuberance (35) which is intended to abut on the capsule of the packing;
- removable connecting means (40) designed to bring the body and the lid relatively closer axially when the box is closed, thus resulting in abutting or pressing the protuberance (35) on the capsule (8) abutting against the jar (3) immobilized in the body (21); and
- at least one opening (50) provided in the body and/or the lid to place the inner volume (17) of the closed box in relation with the ambient medium.

2. The protective box according to claim 1, **characterised in that** the box body comprises a bottom (22) and a peripheral wall (23) defining an open housing (24) for receiving and immobilising a packing.

3. The protective box according to claim 1 or 2, **characterised in that** the box body (21) comprises means (25) for transversely and axially immobilising a packing at a distance from the bottom (22) of the box body.

4. The protective box according to claim 3, **characterised in that** the immobilisation means (25) are formed by at least local excess thicknesses presented by the inner face of the peripheral wall (23) of the box body.

5. The protective box according to claim 1, **characterised in that** box lid (31) is in the form of a cover and comprises a top (32) bordered by a skirt (33) adapted to surround the packing (2) and its capsule (8) at a distance.

6. The protective box according to claim 1 or 5, **characterised in that** the box lid (31) comprises on the inner face of its top (32) a protuberance (35) constituted by an annular rib (36) adapted, when the box is closed, to press the capsule on the jar and to fit this latter in the housing (24) of the body of the box.

7. The protective box according to claim 6, **characterised in that** the annular rib (36) is concentric to the axis of revolution (xx') of the skirt (33) and defines a cavity (37) for fit and protection of a capsule perforator.

8. The protective box according to claim 1, **characterised in that** the connection means (40) are of the type incorporating helicoidal thread and **in that** the top (32) of the lid forms a handle (45) for gripping and rotating the lid with respect to the body.

9. An assembly for sterilising and preserving organic matter or material, **characterised in that** it is composed of a packing (2) comprising a jar (3) and a perforatable capsule (8) for hermetic closure, and of a protective box (20) according to one of claims 1 to 8.

10. The assembly according to claim 9, **characterised in that** the capsule of the packing bears a perforator (10, 11) which is disposed in the cavity (37) for fit and protection defined by the protuberance (35) of the lid of the box.
